(19) Europäisches Patentamt
European Patent Office
Office européen des brevets

(11) **EP 4 473 845 A1**

(12) **EUROPEAN PATENT APPLICATION**
published in accordance with Art. 153(4) EPC

(43) Date of publication:
**11.12.2024 Bulletin 2024/50**

(21) Application number: **22925013.9**

(22) Date of filing: **20.12.2022**

(51) International Patent Classification (IPC):
**A23L 19/00** (2016.01)   **A23B 7/022** (2006.01)

(52) Cooperative Patent Classification (CPC):
**A23B 7/022; A23L 19/00**

(86) International application number:
**PCT/JP2022/046948**

(87) International publication number:
**WO 2023/149109 (10.08.2023 Gazette 2023/32)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **02.02.2022 JP 2022015240**

(71) Applicant: **Nichirei Foods Inc.
Tokyo 104-8402 (JP)**

(72) Inventors:
• **TANABE, Hideya
Sayama-shi, Saitama 350-1331 (JP)**
• **KAMIHIGASHI, Jun
Tokyo 104-8402 (JP)**

(74) Representative: **LLR
2, rue Jean Lantier
75001 Paris (FR)**

(54) **ACEROLA DRY POWDER AND METHOD FOR MANUFACTURING SAME**

(57)    [Problem to be Solved] The present invention provides a method of producing an acerola dry powder for obtaining the acerola dry powder with a high yield.

[Solution to Problem] In the production of an acerola dry powder, acerola juice as a raw material and an alkaline agent containing magnesium oxide are mixed.

**EP 4 473 845 A1**

**Description**

BACKGROUND OF THE INVENTION

Field of the Invention

The present disclosure relates to an acerola dry powder and a method of producing the same.

Background Art

Conventionally, the incorporation of fruit components has been carried out in various types of compositions. In food compositions, for example, the incorporation of fruit components has been carried out for the purpose of improving the flavor thereof. In cosmetic compositions, the incorporation of fruit components has been carried out for the purpose of improving the functionality thereof.

Acerola is known to abundantly contain vitamin C and polyphenols. Since acerola is capable of achieving the effects of preserving the quality and improving the shelf life of processed foods as well as the effect of promoting health and beauty, due to antioxidant effects derived from these components, acerola has conventionally been used as a component in food and beverages as well as cosmetics, expecting such effects. As the use of naturally-derived components, not artificially synthesized components, has gained popularity, particularly with an increasing preference of consumers for health-conscious and natural products, in recent years, it is expected that the demand for acerola, which is a naturally-occurring product that abundantly contains components such as vitamin C and polyphenols, and for processed products thereof, will further increase in the future.

In the case of incorporating acerola to a food, a cosmetic or the like, acerola is incorporated as a processed product (such as juice, juice powder, or the like) of acerola, in general, and various techniques for producing acerola processed products have been attempted. For example, Patent Document 1 discloses a method of obtaining a dry powder of acerola juice.

In the case of obtaining a dry powder of acerola juice, one of the problems is the yield of the dry powder. However, in conventional techniques including the technique disclosed in Patent Document 1, no particular examination or description has been made or given, regarding the production conditions of the dry powder of acerola juice, particularly, the difference in the yield depending on the difference in the type of the alkaline agent to be mixed with the acerola juice.

Citation List

Patent Document

Patent Document 1: U.S. Patent Application Publication No. 2015/327587 A1

SUMMARY OF THE INVENTION

Technical Problem

Under the circumstances described above, a method that improves the yield of the dry powder has been needed, as a method of obtaining a dry powder of acerola juice.

Solution to Problem

The present inventors have found out, in the production of a dry powder of acerola juice, that a high yield of the dry powder can be achieved by undergoing the step of mixing acerola juice, and an alkaline agent containing magnesium oxide. The present disclosure is based on such a finding.

One aspect provides a method of producing an acerola dry powder, the method including a mixing step of mixing acerola juice, and an alkaline agent containing magnesium oxide.

Effects of the Invention

The method of producing an acerola dry powder according to the present disclosure enables to obtain an acerola dry powder with a higher yield as compared to an acerola dry powder obtained by a conventional production method.

The present disclosure provides food safety and security through the supply of an acerola dry powder abundant in natural vitamin C, and plays a part in promoting the achievement of sustainable societies.

BRIEF DESCRIPTION OF THE DRAWINGS

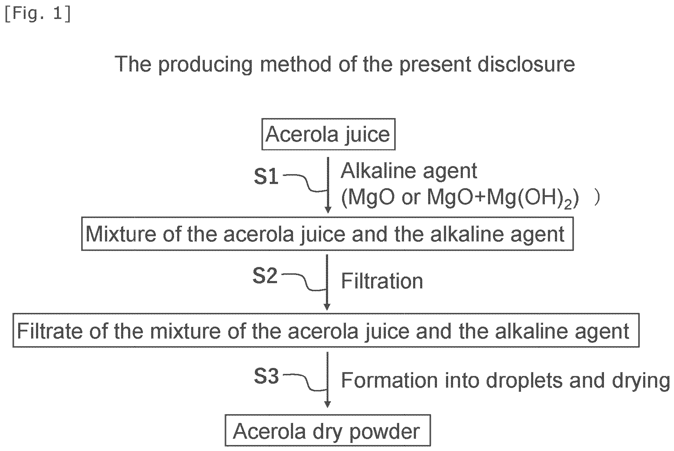

FIG. 1 is a flow chart showing one example of the process of producing the acerola dry powder according to the present disclosure.

DETAILED DESCRIPTION OF THE INVENTION

[Definitions]

In the present disclosure, the "yield of an acerola dry powder" refers to the value of the mass of the resulting acerola dry powder expressed in percentage, with respect to the total mass of the solid content in the acerola juice as a raw material, and the solid content in the alkaline agent to be mixed therewith.
In the present disclosure, the "composition of an acerola dry powder" refers to the components constituting the acerola dry powder and the proportions (contents) thereof. In one embodiment, the expression "an acerola dry powder has a good (or better) composition" refers to the fact that the acerola dry powder contains vitamin C in an amount equal to or higher than that of an acerola dry powder obtained by a conventional production method, or that the contents of the components (such as the alkaline agent) other than vitamin C are lower and the proportion of the solid content in the acerola juice is higher, as compared to those of an acerola dry powder obtained by a conventional production method.
In the present disclosure, the "properties of an acerola dry powder" refer to the color, the moisture value, the hygroscopicity, the storage stability, the particle size distribution and the sphericity of the acerola dry powder. Further, in the present disclosure, the expression "an acerola dry powder has good (or better) properties" refers to the fact that at least one of the properties, namely, at least one of the color, the moisture value, the hygroscopicity, the storage stability, the particle size distribution and the sphericity of the acerola dry powder, is better than that of an acerola dry powder obtained by a conventional production method.
In the present disclosure, the color of an acerola dry powder can be expressed as the brightness of the acerola dry powder. The "brightness" refers to the degree of lightness/darkness of a color, and a higher brightness indicates a lighter color, and a lower brightness indicates a darker color. In other words, a brightness value of 100 indicates white, and a brightness value of 0 indicates black. The brightness is measured using a spectrocolorimeter CM-600d (manufactured by Konica Minolta, Inc.).
The brightness of the acerola dry powder is preferably 76.0 or more. The reasons for setting the threshold value of the brightness of the acerola dry powder to 76.0 are as follows. Since the acerola dry powder according to the present disclosure is capable of achieving the effects of preserving the quality and improving the shelf life as well as the effect of promoting health and beauty, the acerola dry powder may be added to food such as meat, or may be added to a health food such as a tablet. In the case of adding the acerola dry powder to a food or a health food, an acerola dry powder having a higher brightness (namely, closer to 100) is preferred, because the impact inflicted by imparting color on the resulting final product by can be decreased. Further, in the case of forming the acerola dry powder into a product, as it is, it is desirable to decrease the difference of the color of the powder between lots as much as possible. Based on the above, the brightness of the acerola dry powder is preferably set to 76.0 or more, in the present disclosure.
In the present disclosure, the moisture value of the acerola dry powder is calculated as the proportion of the mass of the acerola dry powder which has decreased as compared to that before drying, in cases where from 1.0 to 1.5 g of the acerola dry powder is dried at 105°C for 3 hours. Specifically, when the mass of the acerola dry powder after drying 1.0 g of the acerola dry powder at 105°C for 3 hours, is defined as X g, the moisture value of the acerola dry powder is calculated as the value obtained by the following equation:

$$\text{Moisture value of acerola dry powder} = (1.0 - X) / 1.0 \times 100$$

The moisture value of the acerola dry powder is preferably 7.0% by mass or less. The reason for setting the threshold value of the moisture value of the acerola dry powder to 7.0% by mass is as follows. In general, the moisture value of a dry powder is one of the indices that best reflects the dried state of the dry powder. A dry powder having a low moisture value indicates the fact that a sufficiently dry powder is formed in the production process (such as one using a spray drying technique) thereof. A conventional acerola dry powder produced using a spray drying technique may sometimes have a moisture value of more than 7.0% by mass, and therefore had a problem that the particles of the resulting acerola dry powder aggregate with one another to form masses, during the storage or the like. Based on the above, the moisture value of the acerola dry powder is preferably set to 7.0% by mass or less, in the present disclosure.

[Method of Producing Acerola Dry Powder]

One aspect provides a method of producing an acerola dry powder (hereinafter, also referred to as the "production method according to the present disclosure"). The production method according to the present disclosure includes the step of stirring acerola juice, water if necessary, and an alkaline agent containing magnesium oxide, to evenly mix these components. Water is used for adjusting the concentration of the solid content included in the acerola juice. The production method according to the present disclosure enables to obtain an acerola dry powder (hereinafter, also referred to as the "acerola dry powder according to the present disclosure") having a better composition with a higher yield as compared to an acerola dry powder obtained by a conventional production method.

The acerola juice to be used in the production method according to the present disclosure is one obtained from acerola fruits as a raw material, and it is possible to use any liquid without particular limitation as long as it is a liquid obtained by squeezing, grinding the fruits, or the like. As the acerola juice, it is also possible to use a cloudy juice that contains an insoluble solid content derived from fruit peel and fruit pulp, in addition to a clear juice that does not contain any insoluble solid content derived from fruit peel and fruit pulp.

The content of magnesium oxide in the alkaline agent to be used in the production method according to the present disclosure is preferably from 10 to 100% by mass, more preferably from 20 to 100% by mass, still more preferably from 30 to 100% by mass, particularly preferably from 35 to 100% by mass, and, for example, from 40 to 100% by mass, with respect to the total mass of the alkaline agent.

In one embodiment, the alkaline agent to be used in the production method according to the present disclosure further contains magnesium hydroxide, in addition to magnesium oxide. The content of magnesium hydroxide in the alkaline agent to be used in the production method according to the present disclosure can be set, for example, within the range of from 1 to 90% by mass, from 10 to 80% by mass, from 20 to 60% by mass, or the like.

Magnesium oxide is considered to have a higher reactivity with the components in the juice as compared to that of magnesium hydroxide. Accordingly, as will be shown in Examples to be described later, magnesium oxide allows for adjusting the pH of a mixed solution containing the acerola juice and the alkaline agent closer to an optimal pH by the addition thereof in an amount smaller than that of magnesium hydroxide. As a result, it is thought that the content of vitamin C in the resulting acerola dry powder can be increased.

In another embodiment, the production method according to the present disclosure further includes the step of mixing the acerola juice and an alkaline agent containing magnesium hydroxide. The timing for carrying out the present step is not particularly limited, as long as the effects of the present disclosure can be achieved. The present step may be carried out before the step of mixing the acerola juice and the alkaline agent containing magnesium oxide, or simultaneously with this step. Further, in cases where the present step is carried out simultaneously with the step of mixing the acerola juice and the alkaline agent containing magnesium oxide, each of the alkaline agent containing magnesium oxide and the alkaline agent containing magnesium hydroxide may be independently mixed with the acerola juice, or an alkaline agent obtained by mixing both alkaline agents may be mixed with the acerola juice. In a preferred embodiment, and when the step of mixing the acerola juice and the alkaline agent containing magnesium hydroxide is further included, the alkaline agent obtained by mixing the alkaline agent containing magnesium oxide and the alkaline agent containing magnesium hydroxide, is mixed with the acerola juice.

The amount of the alkaline agent to be mixed with the acerola juice is not particularly limited, as long as the effects of the present disclosure can be achieved. By adding the alkaline agent to the acerola juice to obtain a mixed solution having a pH of from 4 to 6, the effect of facilitating spray drying can be obtained, and in addition, an acerola powder having preferred properties can be obtained. In the case of mixing the alkali juice and the alkaline agent, it is preferred to add the alkaline agent in such an amount that the content of the alkaline agent is from 5 to 15% by mass, more preferably from 6 to 10% by mass, and still more preferably from 6 to 7% by mass, when the total amount of the solid content in the acerola juice and the alkaline agent is taken as 100% by mass. As a result of drying, the solid content in the acerola juice constitutes most of the acerola dry powder. To put it in another way, the content of the alkaline agent is preferably from 5 to 15% by mass, more preferably from 6 to 10% by mass, and still more preferably from 6 to 7% by mass, when the total content of the solid content in the acerola juice and the alkaline agent in the acerola dry powder is taken as 100%.

In a preferred embodiment, the pH of the resulting acerola dry powder is adjusted within the range of from 4 to 6, by mixing the acerola juice and the alkaline agent. By adjusting the pH of the acerola dry powder within such a range, it is possible to obtain an acerola dry powder having good properties such as color (brightness), in addition to achieving a high yield. The pH of the acerola dry powder may be adjusted by changing the mixing ratio of the acerola juice and alkaline agent as appropriate, in the above-described step of mixing the acerola juice and the alkaline agent, may be adjusted by a method acceptable in terms of food hygiene (for example, by adding another component capable of changing the pH, or the like), or may be adjusted by a combination thereof.

The method of mixing the acerola juice and the alkaline agent (and optionally, another component or components) is not particularly limited, as long as the effects of the present disclosure can be achieved, and it is possible to use a method commonly used in the production of food. For example, the mixing can be carried out by stirring and mixing the components described above at room temperature using a commercially available mixing stirrer, by rotating the stirrer at a rotational speed of from 1,000 to 7,500 rpm for 10 to 240 minutes.

In one embodiment, the production method according to the present disclosure includes the step of filtering the above-described mixture of the acerola juice and the alkaline agent. The method of filtering the mixture of the acerola juice and the alkaline agent is not particularly limited, as long as the effects of the present disclosure can be achieved, and it is possible to use a method commonly used in the production of food, such as natural filtration, filtration under reduced pressure, pressurized filtration, centrifugal filtration or diatomaceous earth filtration. The filtration step can be carried out, for example, by filtering the mixture using a commercially available filtration apparatus, and a strainer with a mesh size of from #20 to 400, a membrane filter or a depth filter with an aperture of from 0.2 to 10 μm, or diatomaceous earth.

The method of forming the mixture of the acerola juice and the alkaline agent or a filtrate thereof into a dry powder is not particularly limited, as long as the effects of the present disclosure can be achieved, and it is possible to use a known method such as freeze drying, spray drying, or microwave drying under reduced pressure. In one preferred embodiment, the mixture of the acerola juice and the alkaline agent or a filtrate thereof is formed into droplets using the spray drying method, and then dried. Specifically, the spray drying method is carried out by spraying the mixture of the acerola juice and the alkaline agent or a filtrate thereof into a tower as droplets, and drying the droplets by hot air. The droplets are sprayed using, for example, a pressurized spray nozzle, a rotary spray nozzle, a rotary disk (rotary atomizer), or the like. In the case of using a rotary disk (rotary atomizer), the rotational speed thereof can be preferably set, for example, within the range of from 10,000 to 30,000 rpm. The temperature of the hot air is preferably from 100 to 250°C, more preferably from 110 to 200°C, and still more preferably from 120 to 180°C. Further, the outlet temperature of a drier chamber after cooling is preferably from 20 to 120°C, and more preferably from 50 to 100°C.

One preferred embodiment of the production method according to the present disclosure includes:

(a) the step of mixing acerola juice, and an alkaline agent containing magnesium oxide;
(b) the step of filtering the mixture of the acerola juice and the alkaline agent obtained in the step (a);
(c) the step of forming the filtrate obtained in the step (b) into droplets; and
(d) the step of drying the droplets obtained in the step (c), and collecting the resulting dry powder by a cyclone.

As described above, the production method according to the present disclosure enables to obtain an acerola dry powder with a higher yield as compared to a conventional production method. In addition, the production method according to the present disclosure enables to obtain an acerola dry powder having a good composition.

In the present disclosure, the expression "a high (or higher) yield" used in the context of the method of producing an acerola dry powder refers to the fact that a yield equal to or higher than that obtained by a conventional production method is achieved in the method of producing an acerola dry powder. In one embodiment, a yield of 80% by mass or more is achieved in the production method according to the present disclosure, and a yield of preferably 85% by mass or more, and more preferably 90% by mass or more is achieved.

[Acerola Dry Powder]

The acerola dry powder produced by the production method according to the present disclosure can maintain a high concentration of vitamin C because the amount (mixing proportion) of the alkaline agent to be mixed can be reduced to a low level. Specifically, the acerola dry powder according to the present disclosure has a vitamin C content equal to or higher than that in an acerola dry powder obtained by a conventional production method. In the acerola dry powder according to the present disclosure, the vitamin C content is preferably 34% by mass or more, more preferably 35% by mass or more, still more preferably 35.5% by mass or more, and particularly preferably 36% by mass or more. The higher the vitamin C content in the juice, the better it is, in order to increase the vitamin C content. It is preferred to use acerola juice obtained from an acerola variety (such as NRA309, for example) having a high vitamin C content, and the use of green ripe fruits is also preferred.

The vitamin C content in the acerola dry powder can be measured by high performance liquid chromatography or the indophenol method, in accordance with the Analysis Manual of Japanese Food Composition Table, 2015 edition (seventh edition).

In one preferred embodiment, at least one of the respective properties, namely, at least one of the color (brightness), the hygroscopicity, the storage stability, the particle size distribution and the sphericity of the acerola dry powder according to the present disclosure, is better than that of an acerola dry powder obtained by a conventional production method. In a more preferred embodiment, the color (brightness) of the acerola dry powder according to the present disclosure is better than that of an acerola dry powder obtained by a conventional production method, and in addition, at least one of the respective properties, namely, at least one of the hygroscopicity, the storage stability, the particle size distribution and the sphericity, and particularly preferably all of the respective properties, are better than that of an acerola dry powder obtained by a conventional production method.

The acerola dry powder obtained by the production method according to the present disclosure can be used as it is, or in combination with another material or materials. In one embodiment, the acerola dry powder according to the present disclosure can be used as it is, or alternatively, used as food (a food composition) by being encapsulated in capsules or tableted to be processed into tablets or the like. In another embodiment, the acerola dry powder according to the present disclosure can be used by being incorporated into another food material or food. Since the acerola dry powder according to the present disclosure shows a high vitamin C content, the powder can be used by being suitably incorporated into a food material or food which is required to contain vitamin C.

In one embodiment, the acerola dry powder according to the present disclosure can be used by being incorporated into a cosmetic. Since the acerola dry powder according to the present disclosure shows a high vitamin C content, the powder can be used by being suitably incorporated into a cosmetic which is required to contain vitamin C.

EXAM PLES

The present disclosure will be described more specifically based on the following Examples. However, each aspect of the present disclosure is in no way limited to these Examples.

Preparation Example 1: Preparation of Concentrated Acerola Juice

One hundred kg of acerola fruits were subjected to juice extraction, depulping and concentration to be processed into a clear concentrated juice, and the amount of evaporation residue, namely, the solid content, was adjusted so as to be within the range of from 30 to 45% by mass, to prepare a concentrated acerola juice. Hereinafter, the concentrated acerola juice of the same lot which had been prepared by the procedure described above was used as a raw material, in all Test Plots and Comparison Plots.

Preparation Example 2: Preparation of Acerola Dry Powder - No. 1

The concentrated acerola juice obtained in Preparation Example 1 was divided into six groups, and water and various types of alkaline agents were added thereto so as to achieve the compositions shown in Test Plots 1 to 6 in the following Table 1, followed by stirring and mixing using a T. K. ROMOBIX (manufactured by Tajima KK) (the procedure corresponding to S1 in FIG. 1). For each Plot, the pH after the stirring and mixing, and before filtration, is shown in Table 1. In the above procedure, each alkaline agent is added to the acerola juice having a pH of about 3, to obtain a mixed solution having a pH of from 4 to 6. Note that it has been confirmed that drying the acerola juice without adjusting the pH results in a dried product resembling a solidified candy, and a good acerola dry powder cannot be obtained. Subsequently, the resulting mixture of each group was filtered using a strainer or a glass filter paper (GA-100, manufactured by Advantec Inc.), and the filtrate was collected (the procedure corresponding to S2 in FIG. 1). Thereafter, the resulting filtrate of each group was formed into droplets by the spray method using a rotary spray nozzle, dried with hot air under the conditions of an inlet temperature of hot air of 170°C and an outlet temperature thereof of from 85°C to 91°C, and collected by a cyclone, to obtain each acerola dry powder (the procedure corresponding to S3 in FIG. 1). Meanwhile, water and an alkaline agent (magnesium hydroxide) was added to the concentrated acerola juice obtained in Preparation Example 1, in accordance with Example 1 disclosed in U.S. Patent Application Publication No. 2015/327587 A1, so as to achieve the composition shown in the following Table 1, to obtain an acerola dry powder of Comparison Plot 1, in the same manner. In Table 1, the proportion of the mass of the alkaline agent in each Plot, when the total content of the solid content in the acerola juice and the alkaline agent is taken as 100% by mass, is shown as the amount of the alkaline agent to be mixed with the acerola juice. As the alkaline agent in each of Test Plots 1 to 6, magnesium oxide alone, or a mixture of magnesium oxide and magnesium hydroxide was used. In Comparison Plot 1, magnesium hydroxide alone was used.

[Table 1]

| | | Test Plot 1 | Test Plot 2 | Test Plot 3 | Test Plot 4 | Test Plot 5 | Test Plot 6 | Comparison Plot 1 |
|---|---|---|---|---|---|---|---|---|
| Acerola Solid Content (% by mass) | | 92 | 92 | 91.5 | 91.5 | 90 | 88 | 92 |
| Alkaline Agent (% by mass) | MgO | 8 | 8 | 4.5 | 4.5 | 10 | 12 | 0 |
| | $Mg(OH)_2$ | 0 | 0 | 4 | 4 | 0 | 0 | 8 |
| pH before Filtration | | 5.26 | 5.23 | 5.16 | 5.21 | 5.61 | 5.74 | 4.54 |

(continued)

|  | Test Plot 1 | Test Plot 2 | Test Plot 3 | Test Plot 4 | Test Plot 5 | Test Plot 6 | Comparison Plot 1 |
|---|---|---|---|---|---|---|---|
| Filtration Method | Strainer | Glass Filter Paper | Strainer | Glass Filter Paper | Glass Filter Paper | Glass Filter Paper | Glass Filter Paper |
| Yield (% by mass) | 105.4 | 90.0 | 108.9 | 111.1 | 100.5 | 111.0 | 78.8 |
| Vitamin C Content (% by mass) | 36.7 | 36.7 | 36.4 | 35.9 | 36.5 | 36.6 | 36.3 |
| Color (brightness) | Good | Good | Good | Good | Acceptable | Acceptable | Good |

Example 1: Evaluation of Yield and Vitamin C Content of Acerola Dry Powder - No. 1

The powder yield was calculated for each of the methods of producing an acerola dry powder of Test Plots 1 to 6 and Comparison Plot 1. Further, the vitamin C content of each acerola dry powder was measured using the indophenol method. The results are shown in Table 1.

It can be seen from the results shown in Table 1 that both good powder yield and vitamin C content are achieved in each of the methods of producing an acerola dry powder of Test Plots 1 to 6, which methods include the step of mixing acerola juice, and an alkaline agent containing magnesium oxide, as compared to the method of producing an acerola dry powder of Comparison Plot 1. Further, it can be seen that the methods of Test Plots 1 to 6 enabled to increase the pH of each mixed solution before the filtration with a smaller amount of alkaline agent, and it was easier to adjust the pH within the range of from 4 to 6, which is an optimal range. This is thought to be the effect caused by the fact that magnesium oxide has a reactivity higher than that of magnesium hydroxide.

Example 2: Evaluation of Color (Brightness) of Acerola Dry Powder - No. 1

The color of each of the acerola dry powders of Test Plots 1 to 6 and Comparison Plot 1 was evaluated by visual observation. Those having a brightness equal to or higher than that of Comparison Plot 1 were evaluated as "Good", and those having a brightness lower than that of Comparison Plot 1 were evaluated as "Acceptable". The results are shown in Table 1.

It can be seen from the results shown in Table 1 that each of the acerola dry powders of Test Plots 1 to 4, which has been produced by the method including the step of mixing acerola juice, and an alkaline agent containing magnesium oxide, has a brightness equal to or higher than that of the acerola dry powder of Comparison Plot 1, which has been produced by the method including the step of mixing acerola juice, and an alkaline agent containing magnesium hydroxide. On the other hand, each of the acerola dry powders of Test Plots 5 and 6 had a brightness lower than that of the acerola dry powder of Comparison Plot 1, and had a dark brown color. Therefore, it can be seen that a smaller amount of the alkaline agent to be added is more advantageous from the viewpoint of the color of the dry powder of acerola juice, in the method of producing an acerola dry powder.

The above-described results have shown that the acerola dry powder according to the present disclosure produced using an alkaline agent containing magnesium oxide, has a higher yield and a better composition (higher vitamin C content) as compared to a conventional acerola dry powder produced using an alkaline agent consisting of magnesium hydroxide alone. Further, the results suggested that it is possible to obtain an acerola dry powder which also has good properties such as color (brightness), by adjusting the composition of the alkaline agent, and/or the pH of the mixture of the acerola juice and the alkaline agent.

Preparation Example 3: Preparation of Acerola Dry Powder - No. 2

The concentrated acerola juice obtained in Preparation Example 1 was divided into twelve groups, and water and various types of alkaline agents were added thereto so as to achieve the compositions shown in Test Plots 7 to 18 in the following Table 2, followed by stirring and mixing using a stirrer (Three-One Motor, manufactured by Shinto Scientific Co., Ltd.) (the procedure corresponding to S1 in FIG. 1). For each Plot, the pH after the stirring and mixing, and before filtration, is shown in Table 2. In the above procedure, each alkaline agent is added to the acerola juice having a pH of about 3, to obtain a mixed solution having a pH of from 4 to 6. Note that it has been confirmed that drying the acerola juice without adjusting the pH results in a dried product resembling a solidified candy, and a good acerola dry powder cannot be obtained. Subsequently, the resulting mixture of each group was filtered using a strainer or a glass filter paper (GA-100, manufactured by Advantec Inc.), and the filtrate was collected (the procedure corresponding to S2 in FIG. 1). Thereafter, the resulting filtrate of each group was formed into droplets by the spray method using a rotary spray nozzle, dried with hot air under the conditions of an inlet temperature of hot air of 170°C and an outlet temperature thereof of from 92°C to 98°C, and collected by a cyclone, to obtain each acerola dry powder (the procedure corresponding to S3 in FIG. 1). Meanwhile, the acerola dry powder of Comparison Plot 1 obtained in Preparation Example 2 was prepared as the acerola dry powder of Comparison Plot 2. In Table 2, the proportion of the mass of the alkaline agent in each Plot, when the total content of the solid content in the acerola juice and the alkaline agent is taken as 100% by mass, is shown as the amount of the alkaline agent to be mixed with the acerola juice. As the alkaline agent in each of Test Plots 7 to 18, magnesium oxide or magnesium hydroxide alone, or a mixture of magnesium oxide and magnesium hydroxide was used. In Comparison Plot 2, magnesium hydroxide alone was used.

[Table 2]

| Test Plot | Acerola Solid Content (% by mass) | Alkaline Agent (% by mass) | | Alkaline Agent (total % by mass) | Proportion of Mg(OH)$_2$ in Alkaline Agent(% by mass) | Proportion of MgO in Alkaline Agent (% by mass) | Powder Yield (% by mass) | Powder Yield (Relative Value) | Brightness | Moisture Value (% by mass) | Vitamin C Content (% by mass) | pH | Powder Yield × Vitamin C | |
|---|---|---|---|---|---|---|---|---|---|---|---|---|---|---|
| | | Mg(OH)$_2$ | Mg O | | | | | | | | | | Multiplied Value | Relative Value |
| Test Plot 7 | 94 | 4 | 2 | 6 | 67 | 33 | 106.4 | 92.1 | 79.2 | 7.9 | 35.4 | 4.2 2 | 3766.5 6 | 94.3 |
| Test Plot 8 | 94 | 3 | 3 | 6 | 50 | 50 | 105.6 | 91.4 | 79.3 | 7.1 | 35.2 | 4.2 9 | 3717.1 2 | 93.0 |
| Test Plot 9 | 94 | 2 | 4 | 5 | 33 | 67 | 110.9 | 96.0 | 78.6 | 7.1 | 34.7 | 4.3 9 | 3848.2 3 | 96.3 |
| Test Plot 10 | 94 | 0 | 6 | 6 | 0 | 100 | 115.5 | 100.0 | 78.1 | 6.7 | 34.6 | 4.6 0 | 3996.3 0 | 100.0 |
| Test Plot 11 | 93 | 6 | 1 | 7 | 86 | 14 | 104.9 | 90.8 | 79.3 | 7.0 | 34.7 | 4.3 0 | 3640.0 3 | 91.1 |
| Test Plot 12 | 93 | 5 | 2 | 7 | 71 | 29 | 110.1 | 95.3 | 79.1 | 7.0 | 34.6 | 4.4 1 | 3809.4 6 | 95.3 |
| Test Plot 13 | 93 | 4.5 | 2.5 | 7 | 64 | 36 | 112.1 | 97.1 | 76.9 | 6.9 | 34.2 | 4.5 5 | 3833.8 2 | 95.9 |
| Test Plot 14 | 93 | 4 | 3 | 7 | 57 | 43 | 110.2 | 95.4 | 77.5 | 6.8 | 34.3 | 4.5 1 | 3779.8 6 | 94.6 |
| Test Plot 15 | 93 | 3 | 4 | 7 | 43 | 57 | 111.9 | 96.9 | 78.7 | 6.7 | 34.8 | 4.6 9 | 3894.1 2 | 97.4 |
| Test Plot 16 | 93 | 2 | 5 | 7 | 29 | 71 | 112.3 | 97.2 | 78.0 | 6.9 | 34.8 | 4.8 5 | 3908.0 4 | 97.8 |
| Test Plot 17 | 93 | 1 | 6 | 7 | 14 | 86 | 112.4 | 97.3 | 76.4 | 6.2 | 34.9 | 5.1 3 | 3922.7 6 | 98.2 |
| Test Plot 18 | 93 | 0 | 7 | 7 | 0 | 100 | 113.5 | 98.3 | 75.7 | 6.1 | 34.0 | 5.4 1 | 3859.0 0 | 96.6 |
| Comparison Plot 2 | 94 | 6 | 0 | 6 | 100 | 0 | 55.4 | 48.0 | 79.2 | 7.5 | 35.5 | 3.9 7 | 1966.7 0 | 49.2 |

Example 3: Evaluation of Yield and Vitamin C Content of Acerola Dry Powder - No. 2

The powder yield was calculated for each of the methods of producing an acerola dry powder of Test Plots 7 to 18 and Comparison Plot 2. Further, the vitamin C content of each acerola dry powder was measured using the indophenol method. The yield in Test Plot 10 showing the highest powder yield was taken as 100, and the relative value of the powder yield was determined for each of Test Plots and Comparison Plot. In addition, the relative value of the vitamin C content in each of the acerola dry powders of Test Plots and Comparison Plot was calculated by multiplying the relative value of the powder yield by the vitamin C content. The results are shown in Table 2.

It can be seen from the results shown in Table 2 that each of the acerola dry powders of Test Plots 7 to 18 not only has a stably higher powder yield but also achieves a higher vitamin C content (34% by mass or more), as compared to the acerola dry powder of Comparison Plot 2. In other words, it has been shown that the powder yield, and the total amount of vitamin C in the resulting powder, are both high, in the case of using magnesium oxide alone or using magnesium oxide and magnesium hydroxide in combination as the alkaline agent. In the case of using magnesium hydroxide alone as the alkaline agent, on the other hand, it has been shown that the powder yield is low, as a result of which the total amount of vitamin C in the resulting powder is low.

Based on the results of Test Plot 10 and Test Plot 18, in particular, it can be said that having a content of the alkaline agent of from 6 to 7% by mass is a preferred condition in the case of using magnesium oxide alone as the alkaline agent, from the viewpoints of the powder yield, and the total amount of vitamin C in the resulting powder. Further, based on the results of Test Plots 8 and 9 and 11 to 17, it can be said that having a content of the alkaline agent of from 6 to 7% by mass is a preferred condition in the case of using magnesium oxide and magnesium hydroxide in combination as the alkaline agent, from the viewpoints of the powder yield, and the total amount of vitamin C in the resulting powder. In other words, it can be said that having a content of the alkaline agent of from 6 to 7% by mass is a preferred condition, in either case of using magnesium oxide alone or using magnesium oxide and magnesium hydroxide in combination as the alkaline agent.

While not shown in Table 2, it has been confirmed that the powder yield decreases when the content of the alkaline agent is less than 6% by mass, in either case of using magnesium oxide alone or using magnesium oxide and magnesium hydroxide in combination as the alkaline agent. The reason for this is assumed as follows: when the content of the alkaline agent is less than 6% by mass, the amount of the alkaline agent is insufficient relative to that of the acerola juice, resulting in the occurrence of the acerola juice which did not react with the alkaline agent and remained as it is. Further, it has also been confirmed that the powder yield is unstable, when the content of the alkaline agent is less than 6% by mass.

In addition, while not shown in Table 2, it has been confirmed that the powder yield is not sufficiently improved when the content of the alkaline agent is more than 7% by mass, in either case of using magnesium oxide alone or using magnesium oxide and magnesium hydroxide in combination as the alkaline agent. The reason for this is assumed as follows: when the content of the alkaline agent is more than 7% by mass, the amount of the alkaline agent is excessively high relative to that of the acerola juice. Further, it has also been confirmed that the brightness of the acerola dry powder is low, when the content of the alkaline agent is more than 7% by mass.

Example 4: Evaluation of Color (Brightness) of Acerola Dry Powder - No. 2

The brightness of each of the acerola dry powders of Test Plot 7 to 18 and Comparison Plot 2 was measured by the same method as in Example 2 described above. The results are shown in Table 2.

It can be seen from the results shown in Table 2 that each of the acerola dry powders of Test Plots 7 to 9 and 11 to 17, in which magnesium oxide and magnesium hydroxide were used in combination as the alkaline agent, stably achieved an excellent brightness, which was 76.0 or more. In contrast, the acerola dry powder of Test Plot 18, in which magnesium oxide alone was used as the alkaline agent, had a brightness of less than 76.0.

It can be said from the results of Examples 3 and 4 that, in the case of using magnesium oxide and magnesium hydroxide in combination as the alkaline agent, using these compounds in a total content of from 6 to 7% by mass is a more preferred condition, because it enables to achieve a high brightness (namely, a brightness of 76.0 or more) of the resulting acerola dry powder, in addition to achieving a high yield of the acerola dry powder. Further, it can be said that, in the case of using magnesium oxide and magnesium hydroxide in combination, having a content of magnesium oxide in the alkaline agent of from 14 to 86% by mass (lower limit value: Test Plot 11, upper limit value: Test Plot 17) is a preferred condition.

Example 5: Evaluation of Moisture Value in Acerola Dry Powder

Based on the results shown in Table 2, each of the acerola dry powders of Test Plots 11 to 18 stably achieved a moisture value of 7.0% by mass or less, which is preferred. In other words, either in the case of using magnesium oxide alone or the case of using a mixture of magnesium oxide and magnesium hydroxide, as the alkaline agent, the total content of the alkaline agent is preferably 7% by mass, because it enables to achieve a low moisture value in addition to obtaining a high powder yield.

Example 6: Overall Evaluation of Acerola Dry Powder

Based on the results of Example 4 and Example 5, each of the acerola dry powders of Test Plots 11 to 17 was excellent in all of the powder yield, the brightness and the moisture value. In other words, it can be said that, in the case of using magnesium oxide and magnesium hydroxide in combination as the alkaline agent, having a content of magnesium oxide in the alkaline agent of from 14 to 86% by mass (lower limit value: Test Plot 11, upper limit value: Test Plot 17) is a preferred condition.

In Test Plots 11 to 17, since each of the acerola dry powders of Test Plots 12 to 16 satisfies the conditions of a powder yield of more than 110% by mass, a brightness of more than 76.5 (threshold value: 76.0) and a moisture value of 7.0% by mass or less, it can be said that these are particularly preferred conditions. In other words, it can be said that, in the case of using magnesium oxide and magnesium hydroxide in combination as the alkaline agent, having a content of magnesium oxide in the alkaline agent of from 29 to 71% by mass (lower limit value: Test Plot 12, upper limit value: Test Plot 16) is a preferred condition. In addition, it can be said that using magnesium oxide and magnesium hydroxide in combination as the alkaline agent, and adjusting the mass ratio (magnesium oxide: magnesium hydroxide) of magnesium oxide and magnesium hydroxide in the alkaline agent to 5:9, namely, having a content of magnesium oxide of 36% by mass (Test Plot 13), is one of the most preferred conditions, from the viewpoint of achieving a particularly high relative value of the powder yield.

The above-described results have shown that the acerola dry powder according to the present disclosure produced using an alkaline agent containing magnesium oxide, has a higher yield and a better composition (higher vitamin C content) as compared to a conventional acerola dry powder produced using an alkaline agent consisting of magnesium hydroxide alone. Further, the results suggested that it is possible to obtain an acerola dry powder which also has good properties such as color (brightness), by adjusting the composition of the alkaline agent, and/or the pH of the mixture of the acerola juice and the alkaline agent.

Industrial applicability

According to the present disclosure, it is possible to produce an acerola dry powder having a better composition with a higher yield as compared to an acerola dry powder obtained by a conventional production method.

Supplementary Note

One of the main points of the present disclosure is as follows.

A concentrated acerola juice having a solid content of from 30 to 45% by mass, water and an alkaline agent are added and mixed, to obtain a mixed solution having a pH of from 4 to 6. The filtrate obtained by filtering the resulting mixed solution is dried by the spray drying method, to obtain an acerola dry powder.

Non-concentrated acerola juice may be used instead of the concentrated acerola juice. The use of a concentrated juice is preferred, because the cost of transporting the acerola juice from the harvest area to a powder manufacturing factory and the storage cost at the factory can be reduced. Since the concentrated acerola juice has a high solid concentration and is difficult to be subjected to a treatment such as stirring, water is added thereto to decrease the solid concentration.

Magnesium oxide alone, or a mixture in which magnesium oxide are magnesium hydroxide are combined, is used as the alkaline agent. By adding the alkaline agent to the acerola juice to obtain a mixed solution having a pH of from 4 to 6, the effect of facilitating spray drying can be obtained, and in addition, an acerola powder having preferred properties can be obtained. The alkaline agent is added in such an amount that the content of the alkaline agent is, for example, from 5 to 15% by mass, when the total amount of the solid content in the acerola juice and the alkaline agent is taken as 100% by mass.

[Item 1]

A method of producing an acerola dry powder, the method including a mixing step of mixing acerola juice, and an alkaline agent containing magnesium oxide.

[Item 2]

The method according to item 1, wherein the alkaline agent further contains magnesium hydroxide.

[Item 3]

The method according to item 1 or 2, wherein, in the mixing step, the content of the alkaline agent is from 5 to 15% by mass, when the total amount of the solid content in the acerola juice and the alkaline agent is taken as 100% by mass.

[Item 4]

The method according to item 3, wherein the content of the alkaline agent is from 6 to 7% by mass, when the total amount of the solid content in the acerola juice and the alkaline agent is taken as 100% by mass.

[Item 5]

The method according to item 3, wherein the content of the alkaline agent is 7% by mass, when the total amount of the solid content in the acerola juice and the alkaline agent is taken as 100% by mass.

[Item 6]

The method according to any one of items 1 to 5, wherein the content of magnesium oxide in the alkaline agent is from 14 to 86% by mass.

[Item 7]

The method according to any one of items 1 to 6, wherein the content of magnesium oxide in the alkaline agent is from 20 to 80% by mass.

[Item 8]

The method according to any one of items 1 to 7, wherein the content of magnesium oxide in the alkaline agent is from 29 to 71% by mass.

[Item 9]

The method according to any one of items 1 to 9, wherein the alkaline agent consists of magnesium oxide and magnesium hydroxide.

[Item 10]

The method according to item 9, wherein the mass ratio, magnesium oxide: magnesium hydroxide, of magnesium oxide and magnesium hydroxide in the alkaline agent is 5:9.

[Item 11]

An acerola dry powder produced by the method according to any one of items 1 to 10.

[Item 12]

The acerola dry powder according to item 11, wherein the acerola dry powder is a food composition.

**Claims**

1. A method of producing an acerola dry powder, the method comprising a mixing step of mixing acerola juice, and an alkaline agent containing magnesium oxide.

2. The method according to claim 1, wherein the alkaline agent further contains magnesium hydroxide.

3. The method according to claim 1 or 2, wherein, in the mixing step, the content of the alkaline agent is from 5 to 15% by mass, when the total amount of the solid content in the acerola juice and the alkaline agent is taken as 100% by mass.

4. The method according to claim 3, wherein the content of the alkaline agent is from 6 to 7% by mass, when the total amount of the solid content in the acerola juice and the alkaline agent is taken as 100% by mass.

5. The method according to claim 3, wherein the content of the alkaline agent is 7% by mass, when the total amount of the solid content in the acerola juice and the alkaline agent is taken as 100% by mass.

6. The method according to any one of claims 1 to 5, wherein the content of magnesium oxide in the alkaline agent is from 14 to 86% by mass.

7. The method according to any one of claims 1 to 6, wherein the content of magnesium oxide in the alkaline agent is from 20 to 80% by mass.

8. The method according to any one of claims 1 to 7, wherein the content of magnesium oxide in the alkaline agent is from 29 to 71% by mass.

9. The method according to any one of claims 1 to 8, wherein the alkaline agent consists of magnesium oxide and magnesium hydroxide.

10. The method according to claim 9, wherein the mass ratio, magnesium oxide: magnesium hydroxide, of magnesium oxide and magnesium hydroxide in the alkaline agent is 5:9.

11. An acerola dry powder produced by the method according to any one of claims 1 to 10.

12. The acerola dry powder according to claim 11, wherein the acerola dry powder is a food composition.

[Fig. 1]

## The producing method of the present disclosure

Acerola juice

S1 — Alkaline agent
(MgO or MgO+Mg(OH)$_2$) )

Mixture of the acerola juice and the alkaline agent

S2 — Filtration

Filtrate of the mixture of the acerola juice and the alkaline agent

S3 — Formation into droplets and drying

Acerola dry powder

<table>
<tr><td colspan="2" align="center">**INTERNATIONAL SEARCH REPORT**</td><td colspan="2">International application No.<br>**PCT/JP2022/046948**</td></tr>
</table>

**A.     CLASSIFICATION OF SUBJECT MATTER**

*A23L 19/00*(2016.01)i; *A23B 7/022*(2006.01)i
FI:     A23L19/00 A; A23B7/022

According to International Patent Classification (IPC) or to both national classification and IPC

**B.     FIELDS SEARCHED**

Minimum documentation searched (classification system followed by classification symbols)
A23L19/00; A23B7/02

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS/FSTA/AGRICOLA (STN)

**C.     DOCUMENTS CONSIDERED TO BE RELEVANT**

| Category* | Citation of document, with indication, where appropriate, of the relevant passages | Relevant to claim No. |
|---|---|---|
| A | US 2015/0359254 A1 (DIANA NATURALS) 17 December 2015 (2015-12-17)<br>  claims 1-19, example 1 | 1-12 |
| A | JP 2006-51035 A (ACCESS BUSINESS GROUP INTERNATL LLC) 23 February 2006 (2006-02-23)<br>  example 2 | 1-12 |
| A | GB 2268871 A (BIO-NUTRITIONAL HEALTH SERVICES LIMITED) 26 January 1994 (1994-01-26)<br>  example 1 | 1-12 |

☐ Further documents are listed in the continuation of Box C.        ☑ See patent family annex.

| | |
|---|---|
| *     Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| **03 March 2023** | **14 March 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/046948**

| Patent document cited in search report | | | Publication date (day/month/year) | Patent family member(s) | | | Publication date (day/month/year) |
|---|---|---|---|---|---|---|---|
| US | 2015/0359254 | A1 | 17 December 2015 | WO | 2014/102304 | A1 | |
| JP | 2006-51035 | A | 23 February 2006 | US | 2005/0244518 | A1 | |
| | | | | US | 2004/0131656 | A1 | |
| | | | | US | 2002/0025350 | A1 | |
| | | | | US | 2006/0147563 | A1 | |
| | | | | US | 2008/0226744 | A1 | |
| | | | | WO | 2009/123821 | A2 | |
| GB | 2268871 | A | 26 January 1994 | WO | 1994/001006 | A2 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015327587 A1


**Non-patent literature cited in the description**

- Analysis Manual of Japanese Food Composition Table. 2015